# EUROPEAN PATENT APPLICATION

(11) **EP 4 521 113 A1**
(43) Date of publication of application: **12.03.2025**
(21) Application number: 23205858.6
(22) Date of filing: 25.10.2023
(51) Int. Cl.: G01N 33/53, C12Q 1/6804, C12Q 1/6823

(54) **AFFINITY REAGENT, MARKER AND METHOD FOR ANALYSING A BIOLOGICAL SAMPLE**

(30) Priority: 06.09.2023 EP 23195848
(71) Applicant: Leica Microsystems CMS GmbH, 35578 Wetzlar (DE)
(72) Inventor: Alsheimer, Soeren, 35578 Wetzlar (DE)
(74) Representative: Schaumburg und Partner Patentanwälte mbB

(57) **Abstract**

An affinity reagent (102) for analysing a biological sample is provided, comprising a nucleic acid backbone (106). The affinity reagent (102) further comprises a barcode oligonucleotide (108) attached to the nucleic acid backbone (106). The nucleic acid backbone (106) is configured to specifically bind to a target analyte (114) by its complex structure. The nucleic acid backbone (106) is configured to maintain its complex structure in the presence of the barcode oligonucleotide (108). In a further aspect a marker (100, 206) comprising the affinity reagent (102) and a label (104, 202) is provided. Further, a method for analysing a biological sample with the marker (100, 206) is provided.

## Description

### Technical field

The invention relates to an affinity reagent for analysing a biological sample comprising a backbone and a barcode oligonucleotide. In further aspects, a marker comprising the affinity reagent and a method for analysing a biological sample with the marker are provided.

### Background

Affinity reagents comprise a chemically heterogeneous group and include for example antibodies, nucleic acid probes, and aptamers. Aptamers are typically short nucleic acid sequences that fold into 3-dimensional structures, which are able to bind a target analyte with high affinity and specificity. The use of barcoded affinity reagents such as antibodies, nanobodies, aptamers, or *in situ* probes, which are connected to an oligonucleotide sequence that is unique to said affinity reagent and may contain one or more predetermined sequences that can be hybridised by detectable labels that comprise a complementary barcode, is an effective strategy for cyclical labelling. Such cyclical labelling is used when biological samples shall be analysed for the presence of a high number of target analytes. Apart from labelling barcoded affinity reagents, the barcode may also be directly detected by sequencing.

The function of nucleic acid-based aptamers is based on their correct or specific folding into a complex structure and the maintenance of that complex structure, for example when used as part of a marker in a cyclical labelling method. Since barcoding often uses oligonucleotides, these barcodes may readily interact with nucleic acid aptamers. This may reduce the robustness of nucleic acid-based aptamers, especially when used in cyclical methods.

### Summary

It is an object to provide a barcoded nucleic acid-based affinity reagent and a marker that are robust and a method for analysing a biological sample with the marker.

The aforementioned object is achieved by the subject-matter of the independent claims. Advantageous embodiments are defined in the dependent claims and the following description.

In a first aspect, an affinity reagent for analysing a biological sample is provided, comprising a nucleic acid backbone. The affinity reagent further comprises a barcode oligonucleotide attached to the nucleic acid backbone. The nucleic acid backbone is configured to specifically bind to a target analyte by its complex structure. The nucleic acid backbone is configured to maintain its complex structure in the presence of the barcode oligonucleotide. This enables a robust barcoded affinity reagent with a nucleic acid backbone that provides consistent and specific binding to the target analyte.

The complex structure of the backbone of the affinity reagent may be a secondary, tertiary or quaternary structure of the nucleic acid backbone. Thus, the backbone of the affinity reagent has a secondary, tertiary or quaternary structure, in particular. In particular, the affinity reagent is configured to bind specifically to the target analyte due to the complex structure of the backbone of the affinity reagent. Specifically, individual, discontinuous nucleotides of the nucleic acid of the backbone bind to the target analyte, rather than continuous nucleotides of a sequence of the nucleic acid hybridising to the target analyte, in particular a complementary sequence of the target analyte. This enables binding of the affinity reagent with high specificity.

The barcode oligonucleotide is in particular covalently attached to the backbone of the affinity reagent. The term backbone of the affinity reagent is not limiting to a particular part or element. For example, the backbone of the affinity reagent does not exclusively refer to a phosphate backbone of the nucleic acid. Thus, the barcode oligonucleotide may be attached to any of a phosphate backbone, a pentose sugar, or a nucleobase, or their respective analogues, of the nucleic acid of the backbone of the affinity reagent. In contrast to the backbone of the affinity reagent, the barcode oligonucleotide preferably has no complex structure, instead, the barcode oligonucleotide may have a primary structure configured to hybridise to a complementary oligonucleotide. In particular, the barcode oligonucleotide is configured to hybridise a label oligonucleotide of a detectable label in order to attach the label to the affinity reagent.

For example, in order for the nucleic acid backbone to maintain its complex structure in the presence of the barcode oligonucleotide, the backbone of the affinity reagent and the barcode oligonucleotide may not form a complex structure with each other. In particular, the barcode oligonucleotide and the nucleic acid backbone is the attachment of the barcode oligonucleotide to the nucleic acid backbone.

The biological sample may be a tissue section, for example. Further examples of the biological sample include a cell, a cell culture, an organoid, or a sample of a bodily liquid.

Preferably, the nucleic acid backbone and the barcode oligonucleotide are configured not to hybridise, in particular, in order to maintain the complex structure of the nucleic acid backbone in the presence of the barcode oligonucleotide. In particular, the backbone of the affinity reagent and the barcode oligonucleotide may be configured to not hybridise when at room temperature. This enables avoiding interactions between the barcode oligonucleotide and the nucleic acid backbone of the affinity reagent in order to maintain the complex structure of the nucleic acid backbone in the presence of the barcode oligonucleotide.

In a particularly preferred embodiment, the barcode oligonucleotide has a sequence different to a sequence of the nucleic acid backbone, in particular, in order to maintain the complex structure of the nucleic acid backbone in the presence of the barcode oligonucleotide. For example, any continuous 10 nucleotide sequence of the barcode oligonucleotide is different from a continuous 10 nucleotide sequence of the nucleic acid backbone. This enables avoiding interactions between the barcode oligonucleotide and the nucleic acid backbone of the affinity reagent in order to maintain the complex structure of the nucleic acid backbone in the presence of the barcode oligonucleotide.

Preferably, at least one of the nucleic acid backbone and the barcode oligonucleotide consists essentially of a nucleic acid analogue. In a particular alternative, the other one of the nucleic acid backbone and the barcode oligonucleotide may be a natural nucleic acid. This enables avoiding interactions between the barcode oligonucleotide and the nucleic acid backbone of the affinity reagent in order to maintain the complex structure of the nucleic acid backbone in the presence of the barcode oligonucleotide.

Generally, nucleic acid analogues are compounds which are structurally similar to naturally occurring RNA and DNA. Nucleic acids are chains of nucleotides, which are composed of three parts: a phosphate backbone, a pentose sugar, either ribose or deoxyribose, and one of four nucleobases. An analogue may have any of these altered. The nucleic acid analogue may be an artificial nucleic acid or a xeno nucleic acid, for example. Whereas natural nucleic acids or naturally occurring nucleic acids (DNA and RNA) generally hybridise to each other, in particular, when they are complementary, natural nucleic acids or naturally occurring nucleic acids generally do not hybridise with nucleic acid analogues.

In a particularly preferred embodiment, the nucleic acid analogue comprises at least one of the following, a L-DNA, a nucleic acid backbone comprising phosphorothioate (PT) modifications. This enables avoiding interactions between the barcode oligonucleotide and the nucleic acid backbone of the affinity reagent in order to maintain the complex structure of the nucleic acid backbone in the presence of the barcode oligonucleotide. The L-DNA in particular comprises a L-pentose backbone. For example, in case the backbone of the affinity reagent is a L-DNA and the barcode oligonucleotide is a D-DNA, the backbone of the affinity reagent and the barcode oligonucleotide may not bind to each other and the complex structure of the backbone of the affinity reagent is maintained in the presence of the barcode oligonucleotide. As a further example, the nucleic acid analogue may comprise artificial nucleobases that do not hybridise to natural nucleobases (ATUCG) and therefore preclude hybridisation of the nucleic acid analogue to a natural nucleic acid. Moreover, in another example, both, the backbone of the affinity reagent and the barcode oligonucleotide are nucleic acid analogues that do not bind to each other. In this case, the backbone of the affinity reagent may be a D-DNA and the barcode oligonucleotide may be a PT-modified nucleic acid or the backbone of the affinity reagent and the barcode oligonucleotide may be nucleic acids with different and incompatible sets of natural nucleobases and artificial nucleobases.

A further advantage of the backbone of the affinity reagent being a nucleic acid analogue is that this nucleic acid analogue backbone may maintain its complex structure in the presence of naturally occurring nucleic acids of a biological sample that the affinity reagent may be introduced into.

Preferably, the nucleic acid backbone comprises 10 to 100 nucleotides. This enables particular compact affinity reagents, that are easy and fast to introduce into a biological sample and nevertheless have high specificity to a respective target analyte. In particular, these nucleotides may be nucleotide analogues or artificial nucleotides. Preferably, the backbone comprises 30 to 80 nucleotides. In particular and in a related measure, the backbone may be 10 to 30 kDa in size. In a particular embodiment, the backbone may be multimerised and comprise several copies of the backbone comprising 10 to 100 nucleotides.

Preferably, the barcode oligonucleotide comprises 5 to 25 nucleotides. This enables specific attachment of labels to the affinity reagent, for example. Further, properties of the backbone of the affinity reagent, such as the specific target analyte, may be encoded in the sequence of the barcode oligonucleotide. In a particular embodiment, the affinity reagent may comprise several barcode oligonucleotides that differ in their nucleotide sequence. This enables attachment of multiple labels to the affinity reagent, for example.

Preferably, the nucleic acid backbone is an aptamer. This enables efficiently generating backbones of the affinity reagent that are specific to a range of target analytes, such as various proteins. Aptamers may be generated in various ways, with SELEX (Systematic Evolution of Ligands by EXponential Enrichment) being the most typical route. Additionally, the nucleic acid backbone of the aptamer may preferably comprise modifications, such as glycosylation.

In a further aspect, a marker for analysing a biological sample is provided. The marker comprises the affinity reagent, in particular as described above, and a label comprising a label oligonucleotide and at least one labelling moiety. The label oligonucleotide and the barcode oligonucleotide of the affinity reagent are complementary to each other, and the label is hybridised to the affinity reagent based on complementary base pairing between the label oligonucleotide and the barcode oligonucleotide. In particular, a continuous sequence of the label oligonucleotide binds to a complementary continuous sequence of the barcode oligonucleotide. The marker enables robust and flexible labelling of target analytes in biological samples. In particular, the affinity reagent provides a robust and small means to specifically attach to the target analyte. Via the barcode oligonucleotide a label may be flexibly and specifically attached to the target analyte.

The sequence of the barcode oligonucleotide and the label oligonucleotide may preferably be specific to the respective affinity reagent and/or the respective labelling moiety.

Preferably, the nucleic acid backbone is configured to maintain its complex structure in the presence of the label oligonucleotide. This enables a robust marker.

Preferably, the label oligonucleotide is configured to be sensitive to a degradation agent and the barcode oligonucleotide configured to be resistant to the degradation agent. In particular, the affinity reagent is resistant to the degradation agent. This enables a robust affinity reagent particularly suited for cyclical staining methods.

In particular, this enables selectively degrading the sensitive label oligonucleotide and therefore removing the label from the affinity reagent, in particular from the affinity reagent backbone, by addition of the degradation agent. Preferably, the affinity reagent, in particular the affinity reagent backbone, remains intact in the presence of the degradation agent. In particular, the affinity reagent backbone may continue to be configured to specifically bind to the target analyte in the presence of the degradation agent. Thus, preferably, the affinity reagent backbone is resistant to the degradation agent.

The degradation agent causes degradation only of the sensitive label oligonucleotide, in particular, the degradation agent may digest or fragment the sensitive oligonucleotide. This degradation generally results in loss of function of the oligonucleotide, in particular, the sensitive oligonucleotide no longer binds or is no longer capable of binding to the resistant oligonucleotide. In other words, the sensitive oligonucleotide is configured to be (specifically) degraded by the degradation agent. Preferably, the degradation of the sensitive oligonucleotide is irreversible. Thus, the degradation agent preferably causes the label, in particular the label oligonucleotide, to be removed from the barcode oligonucleotide and render the barcode oligonucleotide accessible to another label in another round of labelling. The specific removal of the label from the affinity reagent whilst leaving the affinity reagent intact enables easy iteration of adding and removing labels to the affinity reagents in cyclical staining methods.

Preferably, the degradation agent is a nuclease. For example, the degradation agent may be a DNase I. This enables easy degradation of the sensitive oligonucleotide, in particular, a naturally occurring nucleic acid, whereas the resistant oligonucleotide may be a nucleic acid analogue.

Preferably, the label oligonucleotide consists essentially of a natural nucleic acid. Whereas natural nucleic acids or naturally occurring nucleic acids (DNA and RNA) are generally sensitive to natural degradation agents such as nucleases, nucleic acid analogues are generally resistant to these degradation agents such as nucleases. Thus, this enables providing an affinity reagent resistant to degradation by natural degradation agents, such as nucleases, and therefore a particular robust affinity reagent. In particular, this enables repeated staining of biological samples based on the affinity reagent by applying degradation agents without causing denaturation or degradation of the affinity reagent. For example, the label oligonucleotide may be a D-DNA. In this case, the backbone of the affinity reagent may preferably be a L-DNA and the barcode oligonucleotide may be a PT-modified D-DNA. In this case, the label oligonucleotide is sensitive to the degradation agent, for example a naturally occurring nuclease and the backbone of the affinity reagent and the barcode oligonucleotide are resistant to the degradation agent.

Preferably, the at least one labelling moiety is optically detectable. This enables easy detection of the marker. For example, the labelling moiety is a fluorophore. The optically detectable labelling moiety may be detected by means of a fluorescence microscope.

Alternatively, the labelling moiety may be an alkyne detectable by Raman spectroscopy, for example.

Preferably, the label comprises a plurality of labelling moieties. The plurality of labelling moieties may all have the same detectable properties or they may have different properties. This enables labels that are particularly easy to detect or labels that are particularly easy to distinguish from each other.

In a further aspect, a method for analysing a biological sample comprising at least a target analyte is provided. The method comprises: introducing into the biological sample at least a first marker, in particular components thereof. The first marker comprising an affinity reagent with a nucleic acid backbone specific to the target analyte and a first label with at least a first labelling moiety. In particular, by introducing the marker, the marker may bind to the respective target analyte. The method further includes generating a first readout of the biological sample with the at least first marker. This step may include directing excitation light onto the biological sample to visualise an optically detectable label and generate a readout from light emitted by the label by means of a microscope, for example. The target analyte may be identified and/or localised within the biological sample based on the first label, in particular the labelling moieties, associated with the target analyte in the readout. The marker enables robust and flexible labelling of target analytes in biological samples. Since the affinity reagent is bound to the target analyte, the first label introduced into the biological sample is associated with that target analyte via the affinity reagent.

Preferably, the method may further include applying a degradation agent to the biological sample in order to remove the first label of the first marker. In particular, this may degrade and release the label oligonucleotide from the marker, in particular, the affinity reagent of the marker. This step may optionally include washing the biological sample in order to remove the released labels. The method may preferably further include introducing at least one second label with at least a second labelling moiety into the biological sample in order to generate a second marker, the second label comprising a second label oligonucleotide configured to hybridise to the barcode oligonucleotide of an or the affinity reagent, and generating a second readout of the biological sample with the second marker. This enables robustly analysing the biological sample, in particular the target analytes. The use of the marker with sensitive and resistant oligonucleotides enables easily degrading specific elements of the marker in order to enable cycling of labels that are associated with the affinity reagent. Preferably, the label oligonucleotides are sensitive to the degradation agent.

The second label oligonucleotide may be identical to the label oligonucleotide of the first label. Thus, the first and second label oligonucleotides may bind to the same barcode oligonucleotide. Alternatively, the affinity reagent may comprise several barcode oligonucleotides that differ in their nucleotide sequence. In this case, the first label oligonucleotide and the second oligonucleotide may have a complementary sequence to a specific one of the several barcode oligonucleotides.

It is particularly preferred that the readout generated is an optical readout, for example, generated by means of a microscope.

Generally, a plurality of markers may be introduced into the biological sample, the markers being specific to respective target analytes, in order to identify a large number of target analytes at the same time.

Preferably, the target analyte is identified and/or localised within the biological sample based on the first label and the second label, in particular the labelling moieties, associated with the target analyte in the first readout and second readout. This enables identifying and/or localising the target analyte with improved precision. Since the affinity reagent is bound to the target analyte, the first and the second label introduced into the biological sample are associated with that target analyte via the affinity reagent.

Preferably, in the step of introducing the at least one first marker, the affinity reagent of the at least one first marker is introduced prior, in particularly separately, to the first label of the first marker, and the affinity reagent is immobilised in the biological sample prior to introducing the first label with the first labelling moiety into the biological sample. This enables introducing the marker into the biological sample separately as smaller individual elements that may penetrate the biological sample easier and then generating the marker in situ. The affinity reagent may be immobilised by crosslinking it with the biological sample, or parts thereof. Preferably, the affinity reagent is immobilised after it has bound to the target analyte. This keeps the affinity reagent in place, which may be particularly beneficial in case a large number of rounds of cyclical labelling is performed subsequently. The label is preferably introduced after crosslinking the affinity reagent.

Preferably, the first labelling moiety and the second labelling moiety have different detectable properties. This enables easily distinguishing between the labelling moieties. For example, the labelling moieties may be fluorophores with different, distinguishable emission wavelengths, excitation wavelengths, or emission duration.

Preferably, after the step of generating the second readout, the steps of applying the degradation agent to degrade the second label oligonucleotide, adding at least one further label with a further labelling moiety, and generating a further readout of the biological sample, are iteratively repeated. This enables robustly analysing the biological sample, in particular the target analytes. In particular, this enables identifying and/or localising the target analyte with improved precision. Particularly in case a biological sample with a large number of target analytes is analysed, each target analyte may be marked with a specific marker and in order to identify and/or localise each target analyte with improved precision. By iteratively removing and attaching different labels to the respective affinity reagents, the target analytes may be identified and/or located with improved precision. The further label preferably has different detectable properties than first and second label, in particular their respective labelling moieties, in order to distinguish the labels.

### Short Description of the Figures

Hereinafter, specific embodiments are described referring to the drawings, wherein:
- Figure 1: is a schematic view of an affinity reagent and a label of a marker,
- Figure 2: is a schematic view of steps of a method for analysing a biological sample with the marker,
- Figure 3a: is a schematic view of an affinity reagent with a miss-folded nucleic acid backbone, and
- Figure 3b: is a schematic view of an affinity reagent with a nucleic acid backbone configured to maintain its complex structure.

### Detailed Description

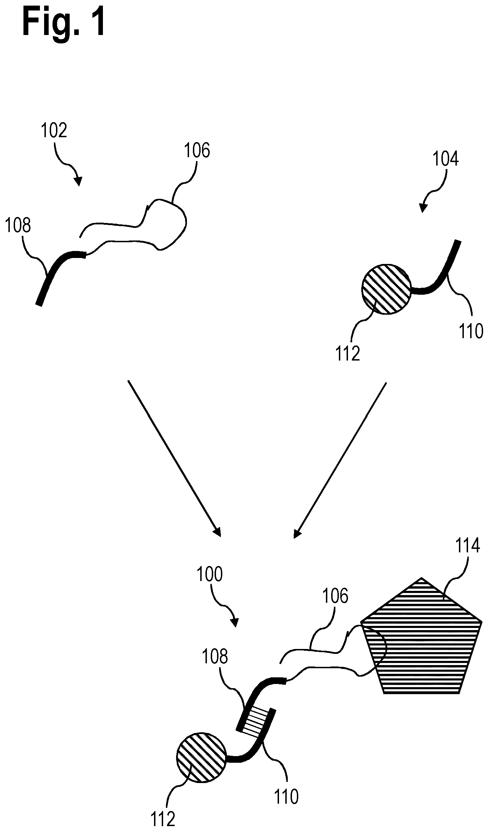

Figure 1 is a schematic view of an affinity reagent 102 and a label 104 of a marker 100. The affinity reagent 102 comprises a nucleic acid backbone 106 and a barcode oligonucleotide 108.

The nucleic acid backbone 106 of the affinity reagent 102 may be a nucleic acid-based aptamer, for example. The barcode oligonucleotide 108 may be covalently attached to the nucleic acid backbone 106 of the affinity reagent 102. The barcode oligonucleotide 108 may be attached to any of a phosphate backbone, a pentose sugar, or a nucleobase, or their respective analogues, of the nucleic acid of the backbone 106 of the affinity reagent 102. For example, attachment of the barcode oligonucleotide 108 may be by an azide/alkyne click chemistry or through NHS coupling. Alternatively, attachment of the barcode oligonucleotide 108 may be by a near-covalent interaction between streptavidin-biotin. To that end, the barcode oligonucleotide 108 may be covalently attached to one of (monovalent) streptavidin and biotin and the nucleic acid backbone 106 of the affinity reagent 102 may be covalently attached to the other one of (monovalent) streptavidin and biotin. When assembling the affinity reagent 102, the streptavidin binds to the biotin resulting in the attachment of the barcode oligonucleotide 108 to the backbone 106.

The nucleic acid backbone 106 of the affinity reagent 102 is configured to specifically bind to a particular target analyte 114, such as a protein of a biological sample. In particular, the nucleic acid backbone 106 may form or fold into a complex structure or conformation. The complex structure of the nucleic acid backbone 106 of the affinity reagent 102 may be a secondary, tertiary and/or quaternary structure of the backbone 106 nucleic acid. Thus, the nucleic acid backbone 106 of the affinity reagent 102 has a secondary, tertiary or quaternary structure, in particular. The affinity reagent 102 binds specifically to the target analyte 114 due to the complex structure of the nucleic acid backbone 106 of the affinity reagent 102. Specifically, individual, discontinuous nucleotides of the nucleic acid of the backbone 106 bind to the target analyte 114. This binding may comprise intermolecular forces such as hydrogen bonding, dipole-dipole interactions, and van der Waals forces. This is in contrast to a nucleic acid hybridising, which is characterised by a continuous sequence of nucleotides of the nucleic acid hybridising to a target analyte, in particular a complementary nucleotide sequence of the target analyte.

The binding of the nucleic acid backbone 106 of the affinity reagent 102 to the target analyte 114 based on the complex structure of the backbone 106 enables particular high affinity binding and specificity to the target analyte 114.

The nucleic acid of the backbone 106 may comprise multiple individual sequences of a nucleic acid or nucleic acid analogue. These individual sequences may bind to each other and form a quaternary structure.

The nucleic acid backbone 106 is configured to maintain its complex structure in the presence of the barcode oligonucleotide 108, in particular. Thus, the barcode oligonucleotide 108 generally does not bind, in particular hybridise, to the nucleic acid backbone 106, except the (covalent) attachment site of the barcode oligonucleotide 108 to the nucleic acid backbone 104. This enables the nucleic acid backbone 106 folding correctly into its complex structure and may specifically bind to the target analyte 114. Specifically, the barcode oligonucleotide 108 generally does not hybridise to the nucleic acid backbone 106 at least at room temperature, or temperatures that staining methods for biological samples are usually carried out at. This may be achieved by selecting a barcode oligonucleotide 108 with a nucleotide sequence that is different to the nucleotide sequence of the nucleic acid backbone 106.

In addition or alternatively, at least one of the nucleic acid backbone 106 and the barcode oligonucleotide 108 of the affinity reagent 102 consists essentially of a nucleic acid analogue.

Generally, nucleic acid analogues are compounds which are structurally similar to naturally occurring RNA and DNA. Nucleic acids are chains of nucleotides, which are composed of three parts: a phosphate backbone, a pentose sugar, either ribose or deoxyribose, and one of four nucleobases. An analogue may have any of these altered. The nucleic acid analogue may be an artificial nucleic acid or a xeno nucleic acid. Specific examples of nucleic acid analogues include: Nucleic acids with modified sugars, such as L-DNA comprising L-pentose, or modification of the ribose at the 2'position with for example 2'-amino, 2'-O-methyl, 2'-fluoro modifications. Further, nucleic acids with modified phosphate backbone such as phosporothioate (PT) or boranophosphate (BP) modifications. These nucleic acid analogues generally do not bind to, in particular hybridise with, naturally occurring nucleic acids, in particular D-DNA comprising D-pentose, or each other. Thus, combinations of naturally occurring nucleic acids and nucleic acid analogues for the nucleic acid backbone 106 and the barcode oligonucleotide 108 may be used that do not readily bind to each other. This avoids the complex structure of the nucleic acid backbone 106 being disrupted by the barcode oligonucleotide 108. This provides a robust affinity reagent that is particularly suited for applications, such as staining, in biological samples.

In a particular preferred embodiment, the nucleic acid backbone 106 consists essentially of a L-DNA and the barcode oligonucleotide 108 consists essentially of a D-DNA, or a PT-modified or BP-modified nucleic acid analogue. The L-DNA of the nucleic acid backbone 106 does not hybridise with either the D-DNA of the barcode oligonucleotide 108. Thus, the barcode oligonucleotide 108 does not disturb the complex structure of the nucleic acid backbone 106 and the nucleic acid backbone 106 maintains its complex structure in the presence of the barcode oligonucleotide 108.

The label 104 comprises a label oligonucleotide 110 and a labelling moiety 112. The label oligonucleotide 110 and the barcode oligonucleotide 108 are complementary to each other such that they may hybridise to each other. Preferably, the barcode oligonucleotide 108 sequence is specific to the nucleic acid backbone 106 of the affinity reagent 102. Thus, the identity of the target analyte 114, which the backbone 106 of the affinity reagent 102 is specific to, may be encoded in the barcode oligonucleotide 108. Similarly, detectable properties of the labelling moiety 112 may be encoded in the barcode oligonucleotide 108. As the consequence, these properties or identities may similarly be encoded in the complementary label oligonucleotide 110.

The labelling moiety 112 is detectable by a particular detection instrument. For example, the labelling moiety 112 may be a fluorophore detectable by a fluorescent microscope.

Similarly to the barcode oligonucleotide 108, the label oligonucleotide 110 may be D-DNA in case the nucleic acid backbone 106 of the affinity reagent 102 is a L-DNA. This avoids the nucleic acid backbone 106 being disturbed by the label oligonucleotide 110 and therefore the nucleic acid backbone 106 also maintains its complex structure in the presence of the label oligonucleotide 110. The D-DNA may hybridise to the complementary D-DNA, or PT-modified or BP-modified nucleic acid analogue of the barcode oligonucleotide 108.

The marker 100 is generated by attaching the label 104 to the affinity reagent 102 based on the complementary barcode oligonucleotide 108 and label oligonucleotide 110.

Optionally, one of the label oligonucleotide 110 and the barcode oligonucleotide 108 is sensitive to a degradation agent whereas the other one of the label oligonucleotide 110 and the barcode oligonucleotide 108 is resistant to the degradation agent. The nucleic acid backbone 106 of the affinity reagent 102 is preferably resistant to the degradation agent. This enables efficiently removing the label 104 from the affinity reagent 102 by adding the degradation agent. For example, in case the label oligonucleotide 110 is sensitive to the degradation agent and the barcode oligonucleotide 108 (as well as the nucleic acid backbone 106) is resistant to the degradation agent, the label oligonucleotide 110 is degraded by the degradation agent resulting in the removal or the dissociation of the label 104 from the affinity reagent 102. To this end, the label oligonucleotide 110 preferably consists essentially of a natural nucleic acid such as D-DNA.

As mentioned above, in a particular preferred embodiment, the nucleic acid backbone 106 consists essentially of a L-DNA and the barcode oligonucleotide 108 consists essentially of a PT-modified or BP-modified nucleic acid analogue. In this case the label oligonucleotide 110 may be a D-DNA and the degradation agent may be a naturally occurring nuclease, such as DNase I. The use of a L-DNA and a PT- or BT-modified nucleic acid analogue for the nucleic acid backbone 106 and the barcode oligonucleotide 108, respectively, has the advantages that (A) both are highly resistant to nucleases and will be stable in a cyclic staining process and (B) that the modified barcode oligonucleotide 108 comprising a D-DNA will not hybridize with an aptamer backbone comprising L-DNA.

In an alternative embodiment, the label oligonucleotide 110 may be a PT-modified nucleic acid analogue sensitive to an iodine degradation agent. The backbone 106 and the barcode oligonucleotide 108 may be an iodine resistant nucleic acid analogue such as L-DNA.

By providing different nucleic acids and/or nucleic acid analogues for the nucleic acid backbone 106, the barcode oligonucleotide 108, and the label oligonucleotide 110, the marker 100 may therefore be particularly robust in terms of its suitability for a cyclical staining process and in terms of its affinity reagent 102, particularly the nucleic acid backbone 106 in the presence of the barcode oligonucleotide 108 and the label oligonucleotide 110.

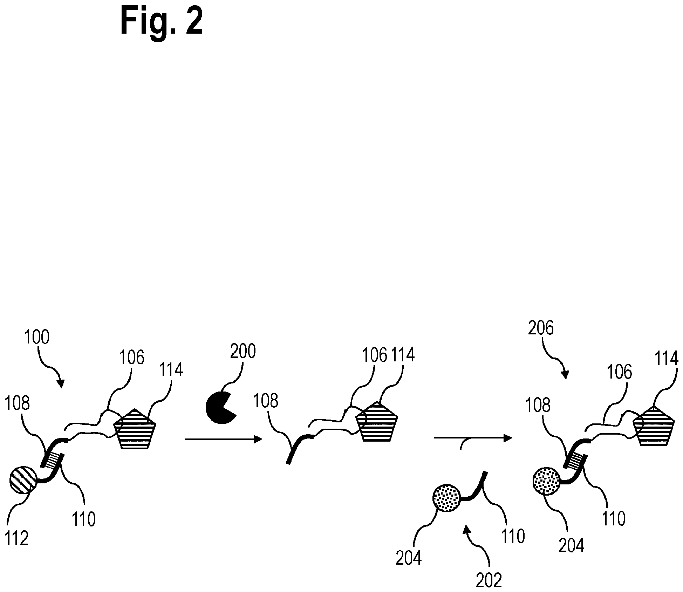

Figure 2 is a schematic view of steps of a method for analysing a biological sample with the marker 100. The marker 100 is initially introduced into the biological sample and binds to the target analyte 114. Similarly, a plurality of markers may be introduced into the biological sample with the plurality of markers having markers that are specific to different target analytes of the biological sample. The labels of the plurality of markers may differ according to their specificity to a particular target analyte. A readout, such as an optical readout, may then be generated of the biological sample. For example, in case the labelling moiety 112 is a fluorophore, the readout may be an optical readout generated by means of a fluorescent microscope. In the readout the detectable label may be detected. The detected label enables identifying and/or localising the target analyte in the biological sample.

The elements of the marker 100 may be introduced into the biological sample individually, such that initially the affinity reagent 102 is introduced into the biological sample and subsequently the label 104 is introduced into the biological sample. Especially in this case, the barcode oligonucleotide 108 is temporarily unbound to the label oligonucleotide 110, which would enable the barcode oligonucleotide 108 to hybridise with a further oligonucleotide. Since the nucleic acid backbone 106 of the affinity reagent 102 is configured to maintain its complex structure in the presence of the barcode oligonucleotide 108, the nucleic acid backbone 106 remains in its complex structure, in which it is configured to specifically bind to the target structure 114. For example, the barcode oligonucleotide 108 may be a PT-modified nucleic acid analogue and the nucleic acid backbone 106 may be a L-DNA analogue.

The further steps are optional to the method. In a next step, a degradation agent 200 may be applied to the biological sample. For example, the degradation agent 200 may a nuclease such as DNase I. The label oligonucleotide 110 is sensitive to the degradation agent 200, for example, due to the label oligonucleotide 110 being a D-DNA. The resistant barcode oligonucleotide 108 and the backbone 106 of the affinity reagent may be a L-DNA resistant to the nuclease degradation agent 200. The digestion of the label oligonucleotide 110 by the nuclease results in the removal of the label 104 from the affinity reagent 102 of the marker 100. Optionally, the elements 110, 112 of the label 104 remaining in the biological sample may be washed out.

In a next step a second label 202 is introduced into the biological sample comprising the label oligonucleotide 110 and a second labelling moiety 204. The label oligonucleotide 110 of the second label 202 readily hybridises to the barcode oligonucleotide 108 of the affinity reagent 102. This generates a second marker 206 in the biological sample attached to the target analyte 114. Preferably, the second labelling moiety 204 has different detectable properties in order to distinguish the first label 102 initially attached to the target analyte 112 via the affinity reagent 102 and the second label 202. In case of fluorophore labelling moieties, the detectable properties may include an excitation wavelength, an emission wavelength and an emission duration. A second readout may be generated of the biological sample with the second marker 206 attached to the target analyte 114. Based on the first and the second readout, the target analyte 114 may be identified and/or localised in the biological sample.

The method may be repeated by removing the second label 202 with the degradation agent 200 and iteratively adding and removing further labels to the biological sample. This enables reliably identifying and/or localising a large number of target analytes with a plurality of markers comprising different labels. In each iteration of the method, a label with particular detectable properties may be assigned to a particular affinity reagent and therefore target analyte. Based on this assignment, the target analyte may be identified and/or localised in the generated readouts.

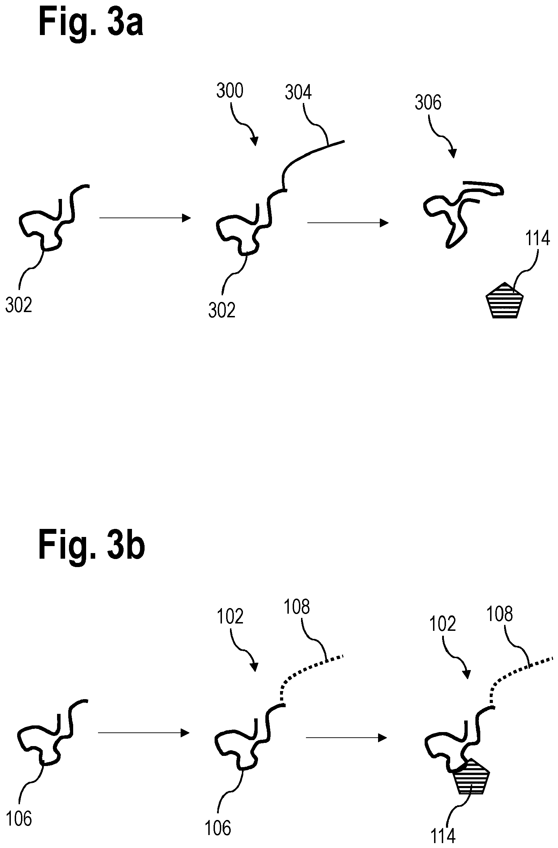

Figure 3a is a schematic view of an affinity reagent 300. The affinity reagent 300 comprises a nucleic acid backbone 302 and a barcode oligonucleotide 304. Both, the nucleic acid backbone 302 and the barcode oligonucleotide 304, are a naturally occurring nucleic acid such as D-DNA. In the left view of the figure, the nucleic acid backbone 302 is correctly folded into its complex structure. Attaching the barcode oligonucleotide 304 to the nucleic acid backbone 302 generates the affinity reagent 300, see the middle view of Fig. 3a. Since the barcode oligonucleotide 304 and the nucleic acid backbone 302 of the affinity reagent 300 are both D-DNA, they may bind to each other. For example, the barcode oligonucleotide 304 and the nucleic acid backbone 302 may at least partially hybridise. This may result in the affinity reagent, in particular the nucleic acid backbone 302, losing its complex structure and a miss-folded affinity reagent 306, in particular in a miss-folded nucleic acid backbone 302, see the right view of Fig. 3a. As a consequence, the miss-folded affinity reagent 306 may not bind to the intended target analyte 114.

Figure 3b is a schematic view of the affinity reagent 102 comprising the nucleic acid backbone 106 of L-DNA and the barcode oligonucleotide 108 of D-DNA. In contrast to the affinity reagent 300 in Figure 3a, the affinity reagent 102, in particular the nucleic acid backbone 106, maintains its complex structure in the presence of the barcode oligonucleotide 108. Consequently, the affinity reagent 102 is able to specifically bind to the target analyte 114.

Identical or similarly acting elements are designated with the same reference signs in all Figures. As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### Reference signs

- 100,206: Marker
- 102: Affinity reagent
- 104,202: Label
- 106: Backbone of affinity reagent
- 108: Barcode oligonucleotide
- 110: Label oligonucleotide
- 112, 204: Labelling moiety
- 114: Target analyte
- 200: Degradation agent
- 300: Affinity reagent
- 302: Nucleic acid backbone of affinity reagent
- 304: Barcode oligonucleotide
- 306: Miss-folded affinity reagent

## Claims

1. An affinity reagent (102) for analysing a biological sample comprising:
a nucleic acid backbone (106), and
a barcode oligonucleotide (108) attached to the nucleic acid backbone (106),
wherein the nucleic acid backbone (106) is configured to specifically bind to a target analyte (114) by its complex structure, and
wherein the nucleic acid backbone (106) is configured to maintain its complex structure in the presence of the barcode oligonucleotide (108).

2. The affinity reagent according to claim 1, wherein the nucleic acid backbone (106) and the barcode oligonucleotide (108) are configured not to hybridise.

3. The affinity reagent according to one of the preceding claims, wherein the barcode oligonucleotide (108) has a sequence different to a sequence of the nucleic acid backbone (106).

4. The affinity reagent according to one of the preceding claims, wherein at least one of the nucleic acid backbone (106) and the barcode oligonucleotide (108) consists essentially of a nucleic acid analogue.

5. The affinity reagent according to claim 4, wherein the nucleic acid analogue comprises at least one of the following, a L-DNA, a nucleic acid backbone comprising phosphorothioate modifications.

6. The affinity reagent according to one of the preceding claims, wherein the nucleic acid backbone (106) comprises 10 to 100 nucleotides.

7. The affinity reagent according to one of the preceding claims, wherein the barcode oligonucleotide (108) comprises 5 to 25 nucleotides.

8. The affinity reagent according to one of the preceding claims, wherein the nucleic acid backbone (106) is an aptamer.

9. A marker (100, 206) for analysing a biological sample comprising:
the affinity reagent (102) according to one of the preceding claims, and
a label (104, 202) comprising a label oligonucleotide (110) and at least one labelling moiety (112, 204),
wherein the label oligonucleotide (110) and the barcode oligonucleotide (108) of the affinity reagent (102) are complementary to each other, and the label (104, 202) is hybridised to the affinity reagent (102) based on complementary base pairing between the label oligonucleotide (110) and the barcode oligonucleotide (108).

10. The marker according to claim 9, wherein the nucleic acid backbone (106) is configured to maintain its complex structure in the presence of the label oligonucleotide (110).

11. The marker according to one of the preceding claims 9 or 10, wherein the label oligonucleotide (110) is configured to be sensitive to a degradation agent (200) and the barcode oligonucleotide (108) configured to be resistant to the degradation agent (200).

12. The marker according to claim 11, wherein the degradation agent (200) is a nuclease.

13. The marker according to one of the preceding claims 9 to 12, wherein the label oligonucleotide (110) consists essentially of a natural nucleic acid.

14. The marker according to one of the preceding claims 9 to 13, wherein the at least one labelling moiety (112, 204) is optically detectable.

15. The marker according to one of the preceding claims 9 to 14, wherein the label (104, 202) comprises a plurality of labelling moieties (112, 204).

16. A method for analysing a biological sample comprising at least a target analyte (114), the method comprising the steps:
introducing into the biological sample at least a first marker (100) according to one of the preceding claims 9 to 15 comprising an affinity reagent (102) with a nucleic acid backbone (106) specific to the target analyte (114) and a first label (104) with at least a first labelling moiety (112), and
generating a first readout of the biological sample with the at least one first marker (100).
